(19)

Europäisches
Patentamt
European
Patent Office
Office européen
des brevets

(11) **EP 2 340 834 A1**

(12) **EUROPEAN PATENT APPLICATION**

(43) Date of publication:
06.07.2011 Bulletin 2011/27

(51) Int Cl.:
*A61K 31/496* (2006.01)      *A61K 9/20* (2006.01)
*A61K 9/28* (2006.01)

(21) Application number: 09180931.9

(22) Date of filing: 30.12.2009

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Abdi Ibrahim Ilac Sanayi Ve Ticaret Anonim Sirketi**
**34555 Istanbul (TR)**

(72) Inventors:
• **Farshi, Farhad**
  **34555, ISTANBUL (TR)**
• **Avci, Recep**
  **34555, ISTANBUL (TR)**
• **Narsisoglu, Nadin**
  **34555, ISTANBUL (TR)**
• **Soylemez, Serdar**
  **34555, ISTANBUL (TR)**
• **Koc, Fikret**
  **34555, ISTANBUL (TR)**

(54) **Enhanced Solubility of Ziprasidone**

(57)    This invention is related to enhanced solubility of ziprasidone free base or pharmaceutically acceptable salt thereof having a mean particle size greater than 85 $\mu$m by using of dividing of disintegrant to be used in internal phase and external

EP 2 340 834 A1

## Description

### Technical Field

The aim of this invention is related to ziprasidone formulations wherein formulations are prepared by internal phase and external phase which provide the enhancement of solubility.

### Background Art

EP 00965343 B (PFIZER PRODUCTS INC.) discloses a composition comprising crystalline ziprasidone free base or crystalline ziprasidone hydrochloride (monohydrate) particles having a mean particle size equal to or less than about 85 $\mu$m.

PCT/WO 2008143960 A (SCIDOSE LLC) delineates a method of enhancing the solubility of ziprasidone or a salt thereof by using some of fatty acids and/or vitamin E.

EP 1703898 A (ALPHARMA, INC.) embraces a ziprasidone formulation wherein the active agent has a mean particle size greater than 85 micrometers.

EP 1753400 A (REDDYS LAB LTD DR) defines a ziprasidone dosage form comprising ziprasidone particles having a mean size at least about 90$\mu$m, and having a ziprasidone bioavailability equal to or greater than the bioavailability of a dosage form where ziprasidone or a salt thereof having a mean size less than 85$\mu$m. It also defines a ziprasidone solid dosage form comprising ziprasidone particles having a mean size at least about 90m and a hydrophilic excipient. Additionally it embraces a solid dosage form comprising ziprasidone hydrochloride particles having a mean size between about 90 $\mu$m and about 180$\mu$m, at least about 90 volume percent of the particles having sizes no greater than about 220$\mu$m, and a sorbitan derivative surfactant in an amount that is about 0.05 to about 5 weight percent of the total dosage form.

PCT/WO 2007102038 A (WOCKHARDT LIMITED) covers a pharmaceutical composition comprising a therapeutically effective amount of ziprasidone hydrochloride hydrate or its salt or pharmaceutically acceptable derivative thereof having a mean particle size preferably in the range from about 100 to about 300 $\mu$m.

PCT/WO 2007126322 A (PLIVAKRAKOW, ZAKLADY FARMACEUTYCZNE S.A.) encompasses a method for obtaining a pharmaceutical composition comprising an active agent ziprasidone to improve solubility and thus bioavailability of the drug, characterized in that the active agent is slugged together with one or more pharmaceutically acceptable excipients.

### Disclosure of Invention

The solubility properties of ziprasidone determine the bioavailability of such drug product. It is a problem in pharmaceutical formulations since solubility problem requires enhancing solubility of ziprasidone by using various methods of pharmaceutical technology.

The subject of this invention is a pharmaceutical composition of ziprasidone or pharmaceutically acceptable salt thereof, which has a mean particle size greater than 85 micrometers and a preparation method of said composition to improve solubility. Thus it exhibits elegant dissolution properties. In this invention pharmaceutical formulations are preferred in tablet form however Zeldox® is capsule. In comparison of tablet against capsule, obtaining of similar or identical dissolution profiles is very difficult. This invention provides tablet formulations having similar dissolution profiles of capsule formulation of Zeldox® .

Another aspect of this invention is a pharmaceutical composition comprises an internal phase and an external phase and in both phases a disintegrant or mixtures of disintegrants are used.

It is advantageous to have disintegrant or mixtures of disintegrants in the internal phase and in the external phase. It was observed that tablets with disintegrants in the internal and the external phase exhibit a better disintegration and a better dissolution profile.

A disintegrant or mixtures of disintegrants are present in both internal and external phases, the dissolution profile of the prepared ziprasidone composition is better than if in the composition disintegrant or mixtures of disintegrants are present only in the external phase alone or only in the internal phase alone.

In order to obtain suitable solubility of ziprasidone having particles greater than 85 micrometers, critical point is dividing the disintegrant or mixtures of disintegrants into the two parts. The first part or initial part is used in internal phase and remaining part is used in external phase.

According to this invention pharmaceutical composition is created by using granulation methods. Granulation method can be wet granulation, dry granulation and the like.

The pharmaceutical composition according to the invention internal phase comprises ziprasidone or pharmaceutically acceptable salt thereof, a part of disintegrant or mixtures of disintegrants and optionally an excipient or excipients are treated together. Treatment can be in the form of wet granulation, dry granulation,direct compression and the like. Remaining disintegrant or mixtures of disintegrants, which are partially used in the internal phase, are incorporated in the external phase.

According to this invention the pharmaceutical composition of ziprasidone or a pharmaceutically acceptable salt thereof comprising gradually disintegrant adding wherein disintegrant or mixture of disintegrants are divided into the at least two lots wherein - an initial part of disintegrant or mixture of disintegrants are used in first phase and - remaining portion of said disintegrant or mixture of disintegrants are used in additional phase or additional phases.

A pharmaceutical composition of ziprasidone wherein it is provided as a combination of initial part of disintegrant or mixture of disintegrants and an external phase containing a second portion of disintegrant or mixture of disintegrants. In particular, half of the disintegrant or mixtures of disintegrants are introduced into the internal phase and the other half is introduced into the external phase.

According to another characteristic of the method, the mixtures of external phase are added to mixtures of internal phase.

According to this invention, the pharmaceutical composition of ziprasidone or pharmaceutically acceptable salt thereof comprises disintegrant, lubricant, binder,diluent/filler,coating agents and the like.

Disintegrants are, but not limited to, sodium starch glycolate, sodium carboxymethyl cellulose, calcium carboxymethyl cellulose, croscarmellose sodium, crospovidone, alginic acid, chitosan, methyl cellulose, microcrystalline cellulose, powdered cellulose,lower alkylsubstituted hydroxypropyl cellulose, polacrilin potassium, starch, pregelatinized starch, sodium alginate,agar, calcium alginate, tribasic calcium phosphate, dibasic calcium phosphate, corn starch, maize starch, waxy maize starch , potato starch, rice starch, carboxymethyl starches, purified wood cellulose, crosslinked carboxymethyl cellulose, low substituted hydroxypropyl cellulose,hydroxylpropyl starch, guar gum, pectins, cation exchange resins, crosslinked homopolymers of vinylpyrrolidone,magnesium silicates, aluminium silicates and colloidal silicon dioxide, mixtures thereof and the like.

Lubricants are, but not limited to, magnesium stearate,calcium stearate,hydrogenated castor oil,glyceryl behenate,glyceryl monostearate,glyceryl palmitostearate,leucine,mineral oil, light mineral oil, myristic acid,palmitic acid, polyethylene glycol,potassium benzoate,sodium benzoate,sodium lauryl sulfate,sodium stearyl fumarate,stearic acid, talc, hydrogenated vegetable oil,zinc stearate, magnesium lauryl sulphate,sodium stearyl fumarate, polyethylene glycol, stearic acid, colloidal silicon dioxide or mixtures thereof and the like. Preferred lubricant is magnesium stearate.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth, sorbitol, acacia, alginates, carrageenan, xanthan gum, locust bean gum and gum arabic, waxes,polyacrylamide , mixtures thereof, and the like.

Diluents/fillers are, but not limited to, mannitol, sorbitol, xylitol, microcrystalline cellulose, silicified microcrystalline cellulose , hydroxypropyl methylcellulose, hydroxypropyl cellulose , pullulan and fast dissolving carbohydrates such as Pharmaburst™, mixtures thereof and the like.

Pharmaceutical composition of ziprasidone comprises; ziprasidone or pharmaceutically acceptable salt is in the range of from about 1 % to about 60 %, binder is in the range of from about 0.1 % to about 20 %, disintegrant is in the range of from about 1 % to about 60 %, diluent/filler is in the range of from about 10 % to about 95 %, lubricant is in the range of from about 0.1 % to about 20 %, by weight of tablet core. In this formulation, it is possible that one or more elements may not be used. In such a case, without some elements, inventors do not aim that such formulation is excluded the invention.

Preferably pharmaceutical composition of ziprasidone comprises; ziprasidone or pharmaceutically acceptable salt is about 18 %, binder is about 2 %, disintegrant is about 22 %, diluent/filler is about 57 %, lubricant is about 1 % by weight of tablet core.

According to the present invention, the pharmaceutical compositions are preferably prepared by a wet granulation process. Thus, for the internal phase, the active principle, the diluent/filler, initial part of disintegrating agent are mixed and then wetted with the granulating liquid. Granulation liquid is prepared by dissolving of binder with a wetting agent. In external phase the mixture of the internal phase, lubricant and remaining portion of binder are mixed. Final mixture is tabletted. Optionally tablets can be coated.

Preferably, the pharmaceutical composition according to the invention internal phase comprises: 1- Wetting agent and binder are mixed until binder is dissolved. 2- Ziprasidon HCl Monohydrate, diluent/filler and half of the disintegrant are mixed in the granulator. 3- The granulation solution (prepared in step 1) is sprayed onto the mixture in the granulator. 4- After the solution is finished, the blend is mixed 5- The mixture is transferred to a dryer and dried. 6- The granules are sieved

The pharmaceutical composition according to this invention external phase comprises, mixtures of internal phase and remaining disintegrant or mixture of disintegrant and optionally an excipient or excipients are mixed and tabletted.

Preferably, according to the invention external phase comprises: 1-Granules of internal phase are transferred to the mixer, half of the remaining disintegrant and additional binder are added to the mixture 2-Lubricant is added to the mixture and mixed 3- The final blend is compressed on a tabletting machine using appropriate punches.

The core tablets can be coated by Opadry II coating solution to produce film coated tablets.

The internal phase of pharmaceutical composition comprises following percentages by weight of total internal phase mixture: ziprasidone or its pharmaceutically acceptable salt is in the range of from about 1 % to about 60 %, binder is in the range of from about 0.1 % to about 20 %, diluent/filler is in the range of from about 10 % to about 95 %, wetting agent is in the range of from about 5 % to about 40 %, initial portion of the disintegrant is in the range of from about 2 % to about 40 % .

Preferably the internal phase of pharmaceutical composition comprises following percentages by weight of total internal phase mixture: ziprasidone or its pharmaceutically acceptable salt is about 18 %, binder is about 2 %, diluent/filler is about 57 %, wetting agent is about 16%, initial portion of the disintegrant is about 7 %.

When wetting agent is evaporated, the external phase of pharmaceutical composition comprises following percentages by weight of total external phase mixture: mixture of external phase is in the range of from about 30 % to about 98 %, remaining binder or mixture of remaining binder and additional binder is in the range of from about 1 % to about 40 %, lubricant is in the range of from about 0.001 % to about 20 %.

When wetting agent is evaporated, the external phase of pharmaceutical composition preferably comprises following percentages by weight of total mixture: mixture of external phase is about 83, 94%, remaining disintegrant or mixture of disintegrants and preferably additional disintegrant are about 15, 08 %, lubricant is about 0.98 %.

Another object of the invention is to achieve bioequivalency to Zeldox® with a ziprasidone or pharmaceutically acceptable salt thereof having particle size is greater than 85 micrometers. In pilot bioequivalence study, formulation according to formulations disclosed in this invention test product as tablet was found bioequivalent to the reference capsule product (Zeldox®).

It is further objective of this invention is to provide a dosage form of ziprasidone or its pharmaceutically acceptable salt thereof selected from, but not limited to tablet, capsule, dispersible tablet, orally disintegrating tablet and the like.

In yet another aspect of this invention is a tablet of ziprasidone or its pharmaceutically acceptable salt thereof characterizing in that the pharmaceutical composition has an in vitro dissolution profile with a similarity factor (f2) of at least 50 to 100 compared to the reference (Zeldox® capsule, Pfizer) dissolution profile.

The similarity factor f2 is a measurement of the similarity as shown in equation 1.

**(Equation 1- Similarity Factor)**

$$f_2 = 50 * \log \frac{100}{\sqrt{1 + \frac{1}{n} \sum_{t=1}^{n} (R_t - T_t)^2}}$$

n: is the number of sampling time points

$R_t$ : is the amount drug released from a *reference* batch at time t

$T_t$: is the amount drug released from a testbatch at time t.

Generally, f2 values greater than 50 ensure sameness of the performance of the reference product and test product.

**Example 1**

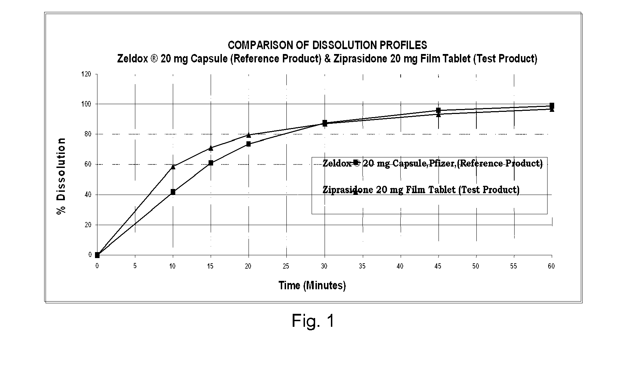

Ziprasidone test tablet is released in 0.05 M Na phosphate buffer, pH 7.5 + 2% SDS (w/w) environment under conditions of 900 ml of a dissolution medium at 37°C $\pm$0.5°C, USP method-ll (pedal), 75 rpm basket speed wherein tablet exhibits a dissolution profile (Figure 1 and Table 1). f2 value is 53,4.

Table 1

| Time (Minutes) | Dissolved % | |
|---|---|---|
| | Zeldox® 20 mg Capsule (Reference) | Ziprasidone 20 mg Film Tablet (Test) |
| 10 | 41,7 | 58,5 |
| 15 | 60,8 | 71,0 |
| 20 | 73,6 | 79,5 |

(continued)

| Time (Minutes) | Dissolved % | |
|---|---|---|
| | Zeldox® 20 mg Capsule (Reference) | Ziprasidone 20 mg Film Tablet (Test) |
| 30 | 87,4 | 87,0 |
| 45 | 95,8 | 93,4 |
| 60 | 98,8 | 96,9 |

**Claims**

1. A pharmaceutical composition, comprising: ziprasidone or pharmaceutically acceptable salt thereof having a mean particle size greater than 85 micrometers and a pharmaceutically acceptable carrier or pharmaceutically acceptable carriers **characterized in that** said pharmaceutical composition is prepared by a method includes at least both internal phase and an external phase.

2. A pharmaceutical composition of ziprasidone or a pharmaceutically acceptable salt thereof having a mean particle size greater than 85 micrometers **characterized in that** said composition is prepared by gradually disintegrant or mixtures of disintegrants adding wherein disintegrant or mixture of disintegrants are divided into the at least two lots wherein - initial portion of disintegrant or mixture of disintegrants are used in internal phase and - remaining portion of disintegrant or mixture of disintegrants, which of some parts used as initial part in internal phase, and optionally additional disintegrant or mixtures of disinegrants are used in external phase.

3. A pharmaceutical composition, comprising: ziprasidone or a pharmaceutically acceptable salt thereof having a mean particle size greater than 85 micrometers and a pharmaceutically acceptable carrier or pharmaceutically acceptable carriers wherein - internal phase comprises ziprasidone or pharmaceutically acceptable salt thereof, a part of disintegrant or mixtures of disintegrants and optionally an excipient or excipients are treated together - remaining disintegrant or mixtures of disintegrants, which are partially used in the internal phase, and optionally additional disintegrant or mixtures of disintegrants are incorporated in the external phase .

4. According to claim 3, half of the disintegrant or mixtures of disintegrants are introduced into the internal phase and the other half and optionally additional disintegrant or disintegrants are introduced into the external phase.

5. A pharmaceutical composition of ziprasidone or a pharmaceutically acceptable salt thereof having a mean particle size greater than 85 micrometers comprises disintegrant, lubricant, binder, diluent/filler and optionally coating agents.

6. According to claim 5, the pharmaceutical composition, comprising: ziprasidone or a pharmaceutically acceptable salt thereof having a mean particle size greater than 85 micrometers and a pharmaceutically acceptable carrier or pharmaceutically acceptable carriers wherein ziprasidone or pharmaceutically acceptable salt is in the range of from about 1 % to about 60 %, binder is in the range of from about 0.1 % to about 20 %, disintegrant is in the range of from about 1 % to about 60 %, diluent/filler is in the range of from about 10 % to about 95 %, lubricant is in the range of from about 0.1 % to about 20 %, by weight of tablet core.

7. According to claim 6, pharmaceutical composition comprises; ziprasidone or pharmaceutically acceptable salt thereof is 18 %, binder is 2 %, disintegrant 22 %, diluent/filler is 57 %, lubricant is 1 % by weight of tablet core.

8. A pharmaceutical composition, comprising: ziprasidone or a pharmaceutically acceptable salt thereof having a mean particle size greater than 85 micrometers and a pharmaceutically acceptable carrier or pharmaceutically acceptable carriers **characterized in that** pharmaceutical composition is prepared in at least two phases wherein - in internal phase, the active principle, the diluent/filler, initial part of disintegrating agent and optionally an excipient or excipients are mixed and optionally granulated, - in external phase, mixtures of internal phase and remaining portion of disintegrant or mixture of disintegrants and optionally additional disintegrant or mixture of disintegrants and optionally an excipient or excipients are mixed

9. According to claim 8, internal phase comprises: a- Wetting agent and binder are mixed until binder is dissolved. b-

Ziprasidon HCl Monohydrate, diluent/filler and ½ of disintegrant are mixed. c- The granulation solution (prepared in step a) is sprayed onto the mixture (obtained in step b) in a granulator. d- After the solution is finished, the blend is mixed e- The mixture is transferred to dryer and dried. f- The granules are sieved external phase comprises: a- Granules of internal phase are transferred to the mixer, ½ remaining disintegrant or mixtures of disintegrants and optionally additional disintegrant or mixtures of disintegrants are added to the mixture, b- Lubricant is added to the mixture and mixed c- The final blend is compressed on a tabletting machine using appropriate punches.

10. A pharmaceutical composition, comprising: ziprasidone or a pharmaceutically acceptable salt thereof having a mean particle size greater than 85 micrometers **characterized in that** internal phase of pharmaceutical composition comprises following percentages by weight of total internal phase mixture: ziprasidone or its pharmaceutically acceptable salt is in the range of from about 1 % to about 60 %, binder is in the range of from about 0.1 % to about 20 %, diluent/filler is in the range of from about 10 % to about 95 %, wetting agent is in the range of from about 5 % to about 40 %, initial portion of the disintegrant or mixtures of disintegrants are in the range of from about 2 % to about 40 % .

11. According to claim 10, internal phase of pharmaceutical composition comprises following percentages by weight of total internal phase mixture:
ziprasidone or its pharmaceutically acceptable salt is 18 %, binder is 2 %,
diluent/filler is 57 %, wetting agent 16%, initial portion of the disintegrant is 7 %.

12. A pharmaceutical composition, comprising: ziprasidone or a pharmaceutically acceptable salt thereof having a mean particle size greater than 85 micrometers **characterized in that** when wetting agent is evaporated, the external phase of pharmaceutical composition comprises following percentages by weight of total external phase mixture: mixture of external phase is in the range of from about 30 % to about 98 %, remaining disintegrant or mixture of disintegrants and optionally additional disintegrant or mixture of disintegrants are in the range of from about 1 % to about 40 %, lubricant is in the range of from about 0.001 % to about 20 %.

13. According to claim 12, when wetting agent is evaporated, the external phase of pharmaceutical composition preferably comprises following percentages by weight of total mixture: mixture of external phase is 83, 94%, remaining disintegrant or mixture of disintegrants and optionally additional disintegrant or mixture of disintegrants are15, 08 %, lubricant is 0.98 %.

14. According to preceding claims disintegrant is selected from the group consisting of sodium starch glycolate, sodium carboxymethyl cellulose, calcium carboxymethyl cellulose, croscarmellose sodium, crospovidone, alginic acid, chitosan, methyl cellulose, microcrystalline cellulose, powdered cellulose,lower alkylsubstituted hydroxypropyl cellulose, polacrilin potassium, starch, pregelatinized starch, sodium alginate,agar, calcium alginate, tribasic calcium phosphate, dibasic calcium phosphate, corn starch, maize starch, waxy maize starch , potato starch, rice starch, carboxymethyl starches, purified wood cellulose, crosslinked carboxymethyl cellulose, low substituted hydroxypropyl cellulose,hydroxylpropyl starch, guar gum, pectins, cation exchange resins, crosslinked homopolymers of vinylpyrrolidone,magnesium silicates, aluminium silicates, colloidal silicon dioxide, mixtures thereof.

15. A tablet of ziprasidone or a pharmaceutically acceptable salt thereof having a mean particle size greater than 85 micrometers **characterizing in that** the pharmaceutical composition has an in vitro dissolution profile with a similarity factor (f2) of at least 50 when compared to the dissolution profile of reference (Zeldox® capsule, Pfizer) product.

16. According to claim 15, dissolution conditions are 0.05 M Na phosphate buffer pH 7.5 + 2% SDS (w/w) and 900 ml of a dissolution medium at 37°C ±0.5°C and USP method-ll (pedal) and 75 rpm speed.

COMPARISON OF DISSOLUTION PROFILES
Zeldox ® 20 mg Capsule (Reference Product) & Ziprasidone 20 mg Film Tablet (Test Product)

Fig. 1

**Europäisches Patentamt**
**European Patent Office**
**Office européen des brevets**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2005/123086 A2 (REDDYS LAB LTD DR [IN]; REDDYS LAB INC DR [US]; VIBHUTHI GOURI SHANKAR) 29 December 2005 (2005-12-29) | 1-6, 8-12,14 | INV.<br>A61K31/496<br>A61K9/20 |
| Y | * page 1, lines 7-9 *<br>* page 4, line 14 - page 5, line 15 *<br>* page 8, lines 6-8 *<br>* examples * | 1-16 | ADD.<br>A61K9/28 |
| | ----- | | |
| X,D | WO 2005/065660 A2 (ALPHARMA INC [US]; BOEHM GARTH [US]; DUNDON JOSEPHINE [US]) 21 July 2005 (2005-07-21) | 1-3,5,8 | |
| Y | * page 2, paragraph 7-9 *<br>* page 10, paragraph 38-39 *<br>* page 14, paragraphs 47,49 *<br>* page 53, paragraph 185 *<br>* page 71, paragraph 0251 - page 72, paragraph 0252 *<br>* page 73, paragraph 254-255 *<br>* page 76, paragraph 266 - page 78, paragraph 173 *<br>* examples 1,8 *<br>* claims 1-3,9-15 * | 1-16 | |
| | ----- | | TECHNICAL FIELDS SEARCHED (IPC)<br><br>A61K |
| Y | WO 2005/107719 A2 (SANDOZ AG [CH]; BARBERA GARY [US]; DOSHI CHETAN CHHABILDAS [US]; PATEL) 17 November 2005 (2005-11-17)<br>* page 1, paragraph 3 *<br>* page 2, paragraph 5 *<br>* page 10, last paragraph *<br>* page 4, paragraph 6 *<br>* examples *<br>* figure 1; table 1 * | 15,16 | |
| | ----- | | |

The present search report has been drawn up for all claims

| Place of search | Date of completion of the search | Examiner |
|---|---|---|
| The Hague | 21 May 2010 | van de Wetering, P |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

& : member of the same patent family, corresponding
 document

EPO FORM 1503 03.82 (P04C01)

EP 2 340 834 A1

## ANNEX TO THE EUROPEAN SEARCH REPORT
## ON EUROPEAN PATENT APPLICATION NO.

EP 09 18 0931

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2010

| Patent document cited in search report | | Publication date | Patent family member(s) | | Publication date |
|---|---|---|---|---|---|
| WO 2005123086 | A2 | 29-12-2005 | EP | 1753400 A2 | 21-02-2007 |
| | | | US | 2007237828 A1 | 11-10-2007 |
| WO 2005065660 | A2 | 21-07-2005 | CA | 2552126 A1 | 21-07-2005 |
| | | | EP | 1703898 A2 | 27-09-2006 |
| WO 2005107719 | A2 | 17-11-2005 | EP | 1744750 A2 | 24-01-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 00965343 B
- WO 2008143960 A
- EP 1703898 A
- EP 1753400 A
- WO 2007102038 A
- WO 2007126322 A